# EUROPÄISCHE PATENTSCHRIFT

(11) **EP 0 313 850 B1**
(45) Veröffentlichungstag und Bekanntmachung des Hinweises auf die Patenterteilung: **13.01.1993**
(21) Anmeldenummer: 88116098.0
(22) Anmeldetag: 29.09.1988
(51) Int. Cl.: C12P 7/40

(54) **Verfahren zur Herstelllung von Brenztraubensäure**
Process for the preparation of pyruvic acid
Procédé de préparation de l'acide pyruvique

(30) Priorität: 01.10.1987 DE 3733157
(43) Veröffentlichungstag der Anmeldung: 03.05.1989
(73) Patentinhaber: BASF Aktiengesellschaft, 67063 Ludwigshafen (DE)
(72) Erfinder: Cooper, Bryan, Dr., D-6800 Mannheim 1 (DE)

(56) Entgegenhaltungen:
- CHEMICAL ABSTRACTS, Band 59, Nr. 11, 25. November 1963, Nr. 13312h, Columbus, Ohio, US; T. SAITON et al.: "Pyruvic acid fermentation by bacteria", & NIPPON NOGEIKAGAKU KAISHI 35, 313-18(1961)
- CHEMICAL ABSTRACTS, Band 63, Nr. 9, 25. Oktober 1965, Nr. 12285b, Columbus, Ohio, US; & JP-A-65 13 793 (KYOWA FERMENTATION INDUSTRY CO., LTD) 02-07-1960

## Beschreibung

Die Erfindung betrifft ein Verfahren zur Herstellung von Brenztraubensäure und deren Salzen.

Brenztraubensäure ist ein wertvolles Zwischenprodukt für chemische Synthesen, z.B. für die Herstellung von Pharmazeutika, Pflanzenschutzmitteln, Polymeren und Lebensmitteln. So ist es verständlich, daß viele Versuche unternommen worden sind, technische Herstellverfahren zu entwickeln. Da Brenztraubensäure ein zentraler Metabolit im Stoffwechsel aller Mikroorganismen ist, verwundert es nicht, daß man sein Interesse in erster Linie auf enzymatische und Fermentationsverfahren gerichtet hat.

Viele der beschriebenen biotechnologischen Herstellverfahren zielen auf die Umwandlung einer geeigneten Kohlenstoffquelle zu Brenztraubensäure. Es ist schon lange bekannt, daß verschiedene Mikroorganismen Brenztraubensäure bilden können. So beschrieben Biquet et al. (Chem. Abstr. 58, 14463 e (1963)) die Bildung der Brenztraubensäure durch verschiedene Pilzstämme der Gattung Candida. Eine ähnliche Beobachtung wurde von Kretovich et al. mit einem Stamm des Pilzes Candida tropicalis gemacht (Chem. Abstr. 62, 6832 a (1965)). Die Verwendung eines Stamms der Art Acetobacter suboxydans zur direkten Herstellung von Brenztraubensäure aus D-Glucose wurde von Abe und Seito (Chem. Abstr. 63, 12285 b (1965)) beschrieben. Die Verwendung von Pilzstämmen der Gattung Mucor zur Herstellung von Brenztraubensäure aus Glucose ist ebenfalls bekannt. (J. Exptl. Botany 16, 487 (1965)). Die Umwandlung von Paraffinen zu Brenztraubensäure und 2-Oxoglutarsäure mit verschiedenen Mycobacterium-Stämmen ist angegeben (Chem. Abstr. 64, 16312 b (1966)). Die Verwendung eines Corynebacterium-Stamms zur Herstellung von Brenztraubensäure aus D-Gluconsäure wurde beschrieben (Chem. Abstr. 70, 86273 w (1969)). Die Umwandlung kurzkettiger Fettsäuren in Brenztraubensäure durch verschiedene Stämme der Gattungen Nocardia, Arthrobacter, Brevibacterium, Corynebacterium, Mycobacterium oder Candida wurde von Maeyashiki und Okada (Chem. Abstr. 82, 123305 d (1975)) angegeben. Eine ähnliche Erfindung unter Verwendung von kurzkettiger Fettsäureamiden mit Stämmen der gleichen Gattungen wurde berichtet (Chem. Abstr. 82, 153737 p (1975)) angegeben. Ein Verfahren zur Herstellung von Brenztraubensäure aus D-Gluconsäure mit den Stämmen Nocardia fumifera bzw. Pseudomonas tabati wurde von Uchio und Hirose (Chem. Abstr. 83, 204815 t (1975)) beschrieben. Ein Verfahren zur Herstellung von Brenztraubensäure aus D-Glucose mit einer Thiamin-und L-Methionin-bedürftigen Mutante von Candida lipolytica wurde von Uchio et al. berichtet (Chem. Abstr. 84, 178212 t (1976)). Ein Verfahren zur Herstellung von Brenztraubensäure aus Glycerin mit einem Stamm der Gattung Xanthomonas campestris wurde von Behrens und Fiedler (DD 135,213 (1979)) angegeben. Die Umwandlung von Zitrusfruchtschalenabfällen in Brenztraubensäure mit Hilfe des Pilzes Debaryomyces coudertii wurde von Moriguchi beschrieben (Agric. Biol. Chem. 46, 955 (1982)). Über die Bildung von Brenztraubensäure aus D-Glucose mit Hilfe des Basidiomyzeten Schizophyllum commune wurde von Takao und Tanida (J. Ferment. Technol. 60, 277 (1982)) berichtet. Ein Verfahren zur Herstellung von Brenztraubensäure aus D-Glucose mit Agaricus campestris wurde von Takao (Chem. Abstr. 98, 15498 r (1983)) beschrieben. Izumi und Matsumura beschrieben Acinetobacter Stämme, die 1,2-Propandiol zu Brenztraubensäure umwandeln (Agric. Biol. Chem. 46, 2653 (1982)). Die Umsetzung von Weinsäure zu Pyruvat mit Pseudomonas putida ist beschrieben worden von Miyata et al. (Chem. Abstr. 104, 205568 d (1986)).

Alle diese Mikroorganismen sind jedoch entweder der Öffentlichkeit nicht zugänglich oder eignen sich nur schlecht für eine technische Produktion, da sie die Brenztraubensäure in nur kleiner Konzentration bzw. geringer Ausbeute, häufig neben einer oder mehreren anderen Säuren, bilden. Dadurch wird eine wirtschaftliche Herstellung nicht möglich.

Es ist ebenfalls beschrieben worden, daß man mit Hilfe von Mikroorganismen razemische Milchsäure oxidativ in Brenztraubensäure überführen kann. Abe und Saito (Chem. Abstr. 58, 5737 d (1963), 6760 (1963)) beschrieben die Verwendung verschiedener, der Öffentlichkeit nicht zugänglicher Bakterienstämme zur Oxidation von razemischer Milchsäure. Diese Stämme eignen sich jedoch schlecht zur Herstellung von Brenztraubensäure, da sie nur in ungenügender Ausbeute (zwischen 27 % und 52 %) das gewünschte Produkt bilden. Außerdem sind erhebliche Mengen Glucose, Pepton und Fleischextrakt notwendig, um die für die Umsetzung notwendige Zellmasse zu erzeugen. Solch teure Mediumsbestandteile verhindern eine wirtschaftliche Herstellung der Brenztraubensäure.

Ein technisches Verfahren zur Herstellung von Brenztraubensäure ist bisher nicht beschrieben worden.

Es bestand also ein Bedarf an einem wirtschaftlichen, technisch ausführbaren Verfahren zur Herstellung von Brenztraubensäure.

Es wurde nun ein Mikroorganismus gefunden, der in der Lage ist, mit hoher Raumzeitausbeute in einem billigen Nährmedium optisch reine D-(-)-Milchsäure quantitativ in Brenztraubensäure zu überführen.

Gegenstand der Erfindung ist ein Verfahren zur Herstellung von Brenztraubensäure und deren Salzen, dadurch gekennzeichnet, daß man das Bakterium Acetobacter spec. ATCC 21409 unter aeroben Bedingungen in Gegenwart von D-(-)-Milchsäure kultiviert.

D-(-)-Milchsäure kann leicht aus Glucose in bekannter Art hergestellt werden.

Acetobacter spec. ATCC 21409 ist bei der American Type Culture Collection (ATCC), Rockeville, Maryland, USA, hinterlegt und frei zugänglich.

Zur Durchführung des erfindungsgemäßen Verfahrens wird der Stamm Acetobacter spec. ATCC 21409 auf ein D-(-)-Milchsäure enthaltendes Nährmedium überimpft und darin inkubiert. Die Fermentation kann kontinuierlich oder diskontinuierlich erfolgen.

Die Zellen des Stamms läßt man direkt auf das Substrat einwirken. Es können beliebige, bekannte Inkubationsverfahren angewendet werden, besonders bevorzugt ist die Verwendung von Fermenten in Form von tiefen, belüfteten und gerührten Tanks. Sehr gute Ergebnisse werden durch Verwendung eines flüssigen Nährmediums erhalten.

Die Wahl des Nährmediums zur Züchtung des Mikroorganismus ist nicht kritisch, doch sollten im Interesse einer erhöhten Wirtschaftlichkeit besonders preisgünstige Mediumsbestandteile verwendet werden. Geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische oder organische Stickstoffverbindungen oder Materialien, die diese Verbindungen enthalten, verwendet werden. Beispiele sind: Ammoniumsalze, Maisquellwasser, Bierhefeautolysat, Sojabohnenmehlhydrolysat, Weizengluten, Hefeextrakt, Hefe, Harnstoff und Kartoffelprotein. Besonders vorteilhaft ist die Verwendung von Maisquellwasser. Als Kohlenstoffquellen können Zucker, wie D-Glucose, Mannose oder Galaktose, Polyalkohole, wie Mannit, oder Alkohole, wie Ethanol, verwendet werden.

Beispiele für anorganische Salze sind die Salze von Calcium, Magnesium, Mangan, Kalium, Zink, Kupfer, Eisen und anderen Metallen. Als Anion der Salze ist besonders das Phosphation zu nennen. Gegebenenfalls werden dem Nährmedium Wachstumsfaktoren zugesetzt, wie z.B. Pantothensäure, p-Aminobezoesäure und Thiamin. Das Mischungsverhältnis der genannten Nährstoffe hängt von der Art der Fermentation ab und wird im Einzelfall festgelegt.

Im allgemeinen eignen sich zur Durchführung des erfindungsgemäßen Verfahrens D-(-)-Milchsäurekonzentrationen von etwa 1 bis 100 g/l, bevorzugt sind Konzentrationen von etwa 10 bis 50 g/l.

Die Züchtungsbedingungen werden so festgelegt, daß die bestmöglichen Ausbeuten erreicht werden. Bevorzugte Züchtungstemperaturen liegen bei 24 bis 32 °C, vorzugsweise bei 26 - 30°C. Der pH-Wert liegt in einem Bereich von 5 - 8, vorzugsweise 6 - 8. Im allgemeinen ist eine Inkubationsdauer von 4 bis 48 Stunden ausreichend. Innerhalb dieser Zeit reichert sich die maximale Menge des gewünschten Produkts im Medium an. Es empfiehlt sich, die Mengen der entstehenden Brenztraubensäure in Inhabationsmedium zu verfolgen und die Reaktion abzubrechen, wenn die Mengen an Brenztraubensäure des Maximum erreicht hat.

Für eine ausreichende Belüftung ist zu sorgen, da die Reaktion nur bei ausreichender Versorgung mit Sauerstoff mit hoher Geschwindigkeit und Ausbeute abläuft.

Die erforderliche Menge D-(-)-Milchsäure kann dem Medium am Anfang auf einmal oder während der Züchtung in mehreren Portionen zugegeben werden.

Die als Substrat genannte D-(-)-Milchsäure wird als Salz in das Nährmedium eingebracht. Es können z.B. die Natrium-, Kalium-, Ammonium- und Calciumsalze der D-(-)-Milchsäure verwendet werden. Besonders vorteilhaft ist die Verwendung des Calciumsalzes.

Die gebildete und ins Medium ausgeschiedene Brenztraubensäure kann nach bekannten Verfahren quantifiziert werden. Vorteilhaft sind zu diesem Zwecke enzymatische Nachweismethoden. Die Brenztraubensäure bzw. deren Salze können nach bekannten Verfahren abgetrennt und gereinigt werden. Hierzu eignen sich z.B. basische Ionenaustauscher. Alternativ hierzu kann die Fermentationsbrühe im Vakuum eingeengt und das Produkt in der Kälte zur Kristallisation gebracht werden. Die so erzeugten Kristalle können durch Zentrifugation abgetrennt und getrocknet werden.

Für den Fall, daß die Brenztraubensäure ohne Aufarbeitung weiter verwendet werden soll, genügt es, die Zellen des Mikroorganismus Acetobacter spec. ATCC 21409 in dem Reaktionsansatz abzutöten, um eine weitere Umsetzung des Produkts zu verhindern. Dieses kann z. B. durch Zugabe von 0,1 % n-Octanol und Erhitzung auf 45 °C für 30 min erfolgen. Alternativ hierzu kann die Fermentationsbrühe mit handelsüblichen Sterilfiltern von dem Mikroorganismus befreit werden. Solche Fermentationsbrühen sollten durch geeignete Maßnahmen, wie z. B. Kühlung, vor mikrobiellen Kontaminationen geschützt werden.

Die folgenden Beispiele erläutern die Erfindung.

### Beispiel 1

### Herstellung einer Stammkultur

Es wurde ein festes Nährmedium (Medium A) hergestellt, welches folgende Bestandteile enthielt:

| | |
|---|---|
| D-(-)-Mannit | 10 g/l |
| Ammoniumsulfat | 5 g/l |
| Magnesiumsulfat-7-hydrat | 0,5 g/l |
| Mangansulfat-1-hydrat | 0,05 g/l |
| Hefeextrakt | 0,05 g/l |
| Pepton | 0,05 g/l |
| Kaliumdihydrogenphosphat | 1,5 g/l |
| Dikaliumhydrogenphosphat | 3,6 g/l |
| Agar | 20 g/l |
| Wasser | ad 1 l. |

Die Phosphatsalze wurden getrennt vom restlichen Medium sterilisiert (20 min bei 121 °C) und nach dem Abkühlen hinzugefügt. Der pH-Wert des Mediums betrug 7.

Zellen des Mikroorganismus Acetobacter spec. ATCC 21409 wurden dezimal verdünnt und auf das feste Nährmedium ausplattiert. Nach 48 h Inkubation bei 28 °C erschienen bei entsprechender Verdünnung einzelne Kolonien. Einzelne Klone wurden abgeimpft und in jeweils 20 ml (in sterilen 100 ml Erlenmeyerkolben) Vorkulturnährmedium (=Medium B) eingeimpft:

| | |
|---|---|
| D-(-)Mannit | 10 g/l |
| Hefeextrakt | 5 g/l |
| Magnesiumsulfat-7-hydrat | 0,5 g/l |
| Wasser ad | 1 l. |

Keine pH-Regulierung, Sterilisation für 20 min bei 121°C.

Die Inkubation dieser Vorkulturen erfolgte bei 28°C in einem handelsüblichen Schüttelinkubator bei 250 UpM für 16 h.

Zur Überprüfung der Biotransformationsleistung wurden je 20 ml des Mediums C in sterile 100 ml Erlenmeyerkolben mit Watteverschluß gefüllt.

| (Medium C: | |
|---|---|
| D-(-)-Mannit | 10 g/l |
| Maisqellwasser | 40 g/l |
| D-(-)-Milchsäure (Calciumsalz) | 10 g/l (berechnet als freie Säure) |
| pH 7 mit 5 M Na0H Sterilisation bei 121 °C für 40 min.) | |

Jeweils 2 ml der Vorkulturen wurden auf dieses Medium überimpft. Die Ansätze wurden bei 28 °C und 250 UpM schüttelnd inkubiert. Die Konzentration an D-(-)-Milchsäure und Brenztraubensäure wurden mit handelsüblichen enzymatischen Testsets bestimmt. Nach 5 h waren in der Regel 100 % des eingesetzten D-Laktats umgesetzt. Die Konzentration an Brenztraubensäure betrug mindestens 10 g/l. Eine diese Leistung aufweisende Kolonie wurde weiterkultiviert und in üblicher Weise lyophilisiert. Dieses Material diente als Impfgut für alle weiteren Experimente.

### Beispiel 2

### Fermentation in Schüttelkolben

100 ml des Mediums C wurden in einen 1000 ml Erlenmeyerkolben mit Watteverschluß gefüllt und sterilisiert. Als Kontrolle wurden 100 ml des gleichen Nährmediums jedoch mit 10 g/l L-(+)-Milchsäure (Natriumsalz, berechnet als Säure) hergerichtet. Beide Kulturen wurden mit 10 ml einer Vorkultur beimpft, wie sie in Beispiel 1 beschrieben wird. Die Inkubation erfolgte bei 28 °C und 250 UpM im Schüttelinkubator. Im Abstand von 60 min wurden Proben entnommen und der Gehalt an Brenztraubensäure bestimmt. Das Ergebnis zeigt die folgende Übersicht.

### Brenztraubensäurekonzentration (g/l)

| Zeit (Stunden) | Ansatz mit Zusatz von D-Laktat | Ansatz mit Zusatz von L-Laktat |
|---|---|---|
| 0,0 | 0,0 | 0,0 |
| 1,0 | 1,2 | 0,8 |
| 2,0 | 3,0 | 1,4 |
| 3,0 | 5,7 | 1,2 |
| 4,0 | 10,2 | 0,9 |
| 5,0 | 11,4 | 0,7 |

Die Analyse der Fermentationsbrühen nach 5 h zeigte, daß die D-(-)-Milchsäure vollständig umgesetzt worden war. Die L-(+)Milchsäure dagegen war noch vollständig vorhanden. Die in diesem Ansatz gebildete Brenztraubensäure rührt von der Oxidation der mit dem Maisquellwasser eingebrachten D-Milchsäure her. Dies erklärt auch die etwas zu hohe Konzentration von Brenztraubensäure in dem Ansatz mit D-(-)-Milchsäure.

### Beispiel 3

### Brenztraubensäurebildung im Fermenter

Ein 1-Liter-Fermenter wurde mit 900 ml des Mediums C, jedoch mit 20 g/l D-Milchsäure (Calciumsalz), beschickt und sterilisiert. Als Vorkultur dienten 100 ml einer 12 h alten, Kultur des Stamms Acetobacter spec. ATCC 21409 auf Medium B, deren Herstellung in Beispiel 1 beschrieben wurde. Die Fermentation erfolgte bei 28 °C mit einer Belüftungsrate von 0,5 vvm und einer Rührgeschwindigkeit von 1000 UpM. Es wurde kein Antischaummittel zugesetzt.

Nach 9 h betrug die Konzentration an Brenztraubensäure (enzymatisch bestimmt) 19,6 g/l.

## Patentansprüche

1. Verfahren zur Herstellung von Brenztraubensäure und deren Salzen, dadurch gekennzeichnet, daß man das Bakterium Acetobacter spec. ATCC 21409 unter aeroben Bedingungen in Gegenwart von D-(-)-Milchsäure kultiviert.

## Revendications

1. Procédé de préparation d'acide pyroracémique et ses sels, caractérisé par le fait que l'on cultive le Bacterium Acetobacter socc. ATCC 21409, dans des conditions aérobies, en présence d'acide D-(-)-lactique.

## Claims

1. A process for the preparation of pyruvic acid and its salts, wherein the bacterium Acetobacter spec. ATCC 21409 is cultivated under aerobic conditions in the presence of D-(-)-lactic acid.
